## Europäisches Patentamt

## European Patent Office

## Office européen des brevets

(11) Veröffentlichungsnummer: **0 004 967**
**A2**

(12) **EUROPÄISCHE PATENTANMELDUNG**

(21) Anmeldenummer: **79101170.3**

(22) Anmeldetag: **17.04.79**

(51) Int. Cl.²: **A 61 N 1/04**

(30) Priorität: **17.04.78 AT 2668/78**
**10.11.78 AT 8068/78**

(43) Veröffentlichungstag der Anmeldung:
**31.10.79 Patentblatt 79/22**

(84) Benannte Vertragsstaaten:
**BE CH DE FR GB IT LU NL SE**

(71) Anmelder: **Mohl, Werner, Dr.Dr.**
**Lazarettgasse 14**
**A-1090 Wien(AT)**

(72) Erfinder: **Mohl, Werner, Dr.Dr.**
**Lazarettgasse 14**
**A-1090 Wien(AT)**

(74) Vertreter: **Kretschmer, Adolf, Dipl-Ing.**
**Schottengasse 3a**
**A-1014 Wien(AT)**

(54) **Einrichtung zum Festlegen eines Sondenkopfes, insbesondere einer Kardialsonde.**

(57) Die Sonde weist zur besseren Verankerung am gewünschten Platz ihrer Anbringung über den Sondenkopf (1) vorragende ausfahrbare Verankerungselemente (4) auf. Zur Erzielung einer sicheren Verankerung werden die Verankerungselemente (4) von einem Energiewandler oder einem Energiespeicher (11) angetrieben, dessen Betätigung von einer Stelle außerhalb des Sondenkopfes (1) möglich ist. Als Energiespeicher kommen in erster Linie Federn in Betracht, während ein Energiewandler beispielsweise von einem Elektromagneten gebildet sein kann.

FIG. 1

- 1 -

**Einrichtung zum Festlegen eines Sondenkopfes, insbesondere einer Kardialsonde.**

Die vorliegende Erfindung bezieht sich auf eine Einrichtung zum Festlegen eines Sondenkopfes, insbesondere für Kardialsonden, bei welcher vorzugsweise von nadelförmigen, gebogenen oder abgewinkelten Teilen gebildete Verankerungsteile mechanisch in ihre über den Außendurchmesser des Sondenkopfes vorragende Verankerungslage am gewünschten Ort bringbar sind. Es sind bereits Einrichtungen bekannt, bei welchen über den Sondenkopf vorragende Nylondrähte vorgesehen sind, welche unter Vermittlung eines Mandrins in ihre über den Sondenkopf vorragende Verankerungslage vorgeschoben werden. Der Innendurchmesser solcher bekannter Sonden beträgt üblicherweise weniger als 1 mm und der Außendurchmesser der Sondenzuleitung liegt in der Größenordnung von 2 mm. Die vom Mandrin auf die Verankerungsteile ausübbare Kraft ist daher durch die Elastizität und Flexibilität des Materials begrenzt und es besteht kaum eine Möglichkeit zu überprüfen, ob die Verankerungselemente tatsächlich in eine Verankerungsstellung gelangt sind oder nicht. Die sichere Verankerung eines solchen Sondenkopfes für Kardialsonden ist aber deshalb von wesentlicher Bedeutung, da die Sonde nur dann wirksam ist, wenn die Elektrode an einer geeigneten Stelle des Myocards angreift. Wenn eine Dislokation des Sondenkopfes auftritt, ist die Funktion ernstlich in Frage gestellt, da in aller Regel die Reizschwelle ansteigen wird und somit eine wirksame Stimulation des Herzmuskels nicht mehr erfolgt. Derartige Sonden können nun sowohl transvenös als auch im Zuge einer Herzoperation epimyokardial angebracht werden, wobei die Gefahr einer Dislokation des Sondenkopfes in beiden Fällen die gleichen Probleme mit sich bringt.

Die Erfindung stellt sich nun die Aufgabe, eine Einrichtung der oben bezeichneten Art zu schaffen, welche eine hohe Dislokationssicherheit aufweist und deren Positionierung mit einfachen Mitteln, d.h. ohne komplizierte Manipulation des Mandrins, deren Effekt fragwürdig ist, möglich ist. Zur Lösung dieser Aufgabe ist die Erfindung im wesentlichen dadurch gekennzeichnet, daß die Verankerungsteile mit einem Energiewandler oder einem Energiespeicher zusammenwirken, welcher von einer Stelle außerhalb des Sondenkopfes betätigbar ist. Auf diese Weise werden alle Unsicherheiten, welche sich durch eine lediglich mechanische Übertragung des Verschiebeweges der Verankerungsteile durch einen Mandrin ergeben, vermieden und es wird eine sichere Verankerung ermöglicht, da die für die Verschiebung der Verankerungsglieder in ihre Verankerungslage erforderlichen Kräfte unmittelbar dort angreifen, wo sie mit hoher Sicherheit auch zur Wirkung gelangen können. Alle Unsicherheiten, welche sich durch eine mechanische Kraftübertragung über die gesamte Länge der Sondenzuleitung ergeben würden, werden auf diese Weise vermieden.

Die Ausbildung ist hiebei vorzugsweise so getroffen, daß die Verankerungsteile mit einem Energiespeicher verbunden sind, wobei der vor der Energieabgabe bestehende Zustand des Energiespeichers der eingezogenen Ausgangslage der Verankerungsteile und der nach der Energieabgabe bestehende Zustand des Energiespeichers der Verankerungslage derselben entspricht und zur Abgabe der Energie des Energiespeichers ein Betätigungsglied vorgesehen ist, welches von einer Stelle außerhalb des Sondenkopfes betätigbar ist. Durch die Verwendung des Energiespeichers ergibt sich gegenüber Ausbildungen, bei welchen die mechanische Kraftübertragung über Mandrins für die Positionierung der Verankerungsteile verwendet wird, der Vorteil, daß die Verankerungsteile rasch in ihre Verankerungslage ohne nennenswerte Beanspruchungen der Sondenzuleitung und des Sondenkopfes selbst gebracht werden. Dieses rasche Eindringen der Verankerungslage in das Herzmuskel-Gewebe erlaubt eine größere Verankerungslänge, da auch längere Verankerungsteile sicher aus dem Sondenkopf ausgefahren werden können.

In einfacher Weise kann erfindungsgemäß der Energiespeicher von einer Sprengpatrone und das Betätigungsglied von einer Zündleitung gebildet sein.

Gemäß einer weiteren bevorzugten Ausbildung der Erfindung ist die Anordnung so getroffen, daß der Energiespeicher von einer Feder gebildet ist, welche durch ein lösbares Verriegelungselement in ihrer gespannten Lage festlegbar ist.

Das Verriegelungselement kann hiebei in besonders einfacher Weise von einem Haken gebildet sein. In diesem Fall ist das Betätigungsglied von einem Zugglied gebildet, mit welchem der Haken aus seiner Eingriffslage ausklinkbar ist. Alternativ ist es jedoch möglich, die Ausbildung so zu treffen, daß das Verriegelungselement von einem leicht schmelzenden Zugglied, insbesondere einer Lötstelle gebildet ist, wobei dann das Betätigungsglied von den elektrischen Zuleitungen für das Schmelzen des Zuggliedes gebildet ist. Als Feder kann sowohl eine Schraubenfeder als auch eine ebene Spiralfeder bevorzugt verwendet werden, wobei die ebene Spiralfeder den Vorteil mit sich bringt, daß der Austrittswinkel der Verankerungsteile aus dem Sondenkopf relativ zu einer den Sondenkopf tangierenden Radialebene besonders klein gewählt werden kann, wodurch eine große Verankerungsbreite ermöglicht wird. Derartig kleine Austrittswinkel, welche eine große Verankerungsbreite ermöglichen, sind nur unter Zuhilfenahme eines Kraftspeichers, nicht aber unter Verwendung des Mandrins zum Zwecke des Ausschiebens von Verankerungsteilen möglich, da in letzterem Fall der zur Verfügung stehende Hebelarm für das Ausfahren der Verankerungsteile überaus ungünstig ist und auf Grund der Elastizität der verwendeten Materialien eine solche Ausbildung nicht realisierbar ist.

Bei Verwendung von Schraubenfedern oder Spiralfedern als Energiespeicher sind die Verankerungsteile vorzugsweise an der an das Verriegelungselement anschließenden Windung der Feder festgelegt, wodurch sich ein großer Ausfahrweg für die Verankerungsteile ergibt und eine große Verankerungsbreite ermöglicht wird.

Die Ausbildung kann aber auch im Rahmen der Erfindung so getroffen sein, daß der Energiespeicher von einem unter Gasdruck stehenden Zylinder eines Zylinder-Kolbenaggregates gebildet ist, dessen Kolben von einer Feder belastet ist, wobei das Betätigungsglied vorzugsweise von einem Zugglied gebildet ist, welches an einem Verschlußteil des Zylinders angreift, und die Verankerungsteile mit dem Kolben oder einer Kolbenstange verbunden sind. In diesem Fall genügt es, mit dem Betätigungsglied beispielsweise einen eine Öffnung des Zylinders abdeckenden, auf den Zylinder aufgeklebten Verschlußteil aufzureißen. Wenn sich in einem solchen Fall nur eine kleine Ausströmöffnung für das Gas im Zylinder ergibt, wird die Austrittsgeschwindigkeit der Verankerungsteile zwar geringer sein, jedoch kann die Kraft der Feder in einem solchen Fall sehr hoch bemessen sein, wodurch ein langsames, aber dafür sehr weit reichendes Eindringen der Verankerungsteile in das Muskelgewebe ermöglicht wird. Ein solches langsames Eindringen verringert die Traumatisationsgefahr des an den Sondenkopf anschließenden Muskelgewebes und ermöglicht eine besonders schonende sichere Festlegung des Sondenkopfes.

Es hat sich nun gezeigt, daß in vielen Fällen eine einmal gewählte Positionierung eines Sondenkopfes keine befriedigenden Ergebnisse mehr ergibt. In solchen Fällen muß daher die Möglichkeit gegeben werden, den bereits verankerten Sondenkopf mit einfachen Mitteln wieder aus seiner Verankerungslage herauszubewegen, wobei die in diesem Falle auftretende Beschädigung, beispielsweise Myocards bei einer Kardialsonde, so gering wie nur möglich gehalten werden soll. Dadurch, daß die Verankerungsteile vorzugsweise mit einem Anker eines als Elektromagnet ausgebildeten Energiewandlers verbunden sind und die elektrischen Leitungen zur Versorgung des Elektromagneten in der Sondenzuleitung untergebracht sind, ergibt sich gleichfalls der Vorteil, daß die Verschiebungskräfte, welche das Ausfahren der Verankerungsteile ermöglichen sollen, unmittelbar dort auftreten, wo die Verschiebung erwünscht ist, wobei die Unsicherheiten, welche sich durch die Elastizität des relativ dünnen Mandrins ergibt, vermieden werden. Die Verwendung eines Elektromagneten erlaubt darüberhinaus eine Umkehr der Bewegungsrichtung und damit sowohl ein Ausfahren der Verankerungsteile, als auch ein Einfahren der Verankerungsteile. In besonders einfacher Weise kann die elektrische Versorgung des Elektromagneten über die elektrische Zuleitung zum Sondenkopf, über welche auch das Reizpotential eingebracht wird, und über den Mandrin erfolgen, sodaß überhaupt keine zusätzlichen elektrischen Leitungen zur Versorgung des Elektromagenten erforderlich sind.

Wenn, wie es einer bevorzugten Ausbildung der erfindungsgemäßen Einrichtung entspricht, sowohl die Ausgangslage als auch die Endlage der Verankerungsteile verriegelbar ist, ergibt sich eine besonders gute Sicherung der eingezogenen sowie der ausgefahrenen Lage. Dies ist insbesondere deshalb von besonderer Bedeutung, da die Sonde bei eingezogenen Verankerungsteilen verschoben werden soll um Verletzungen des Gewebes während des Verschiebens zu vermeiden. Andererseits soll die einmal gewählte ausgefahrene Lage, sobald kein weiterer Anlaß mehr für eine Veränderung der Lage des Sondenkopfes besteht, gesichert sein, um ein unbeabsichtigtes Lösen der Verankerung zu vermeiden.

Für diese Sicherung der jeweiligen Lage kann gemäß einer bevorzugten Ausführungsform die Ausbildung so getroffen sein, daß die Verankerungsteile federnd in einer der Verankerungsstellung entsprechenden Stellung und/oder in einer der eingezogenen Ausgangslage entsprechenden Stellung gehalten sind. Die magnetischen Kräfte treten lediglich bei Anlegen von Strom an elektrischen Spulen auf und da die Verschiebung nur relativ kurze Zeit beansprucht, können diese Spulen ohne weiteres beträchtlich überlastet werden, ohne daß es zu einer Zerstörung der Spulen kommt.

Die Verschiebung des Ankers kann in einfacher Weise dadurch bewirkt werden, daß in Achsrichtung des Ankers zwei Spulen in Abstand voneinander angeordnet sind, wobei der Anker einen weichmagnetischen Abschnitt aufweist, welcher in der Verankerungslage von der einen der beiden Spulen, und in der zurückgezogenen Lage der Verankerungsteile von der anderen der beiden Spulen umschlossen ist. In diesem Fall können drei elektrische Leiter an den Ursprung der Kardialsonde herausgeführt sein, wobei ein Anschluß jeweils mit einem Anschluß beider Spulen verbunden ist, und die beiden anderen Anschlüsse zu jeweils einer der beiden Spulen führen. Auf diese Weise kann durch Wahl von jeweils zwei der drei Leiter eine der beiden Spulen erregt werden, und die Verschiebung des weichmagnetischen Abschnittes des Ankers in das Innere der erregten Spule bewirken. Die Ausbildung kann aber auch so getroffen sein, daß nur zwei Leiter herausgeführt sind, wobei die beiden Spulen unter Zwischenschaltung von zwei Dioden miteinander verbunden sind, wobei der eine Anschluß zwischen diesen beiden Dioden angeschlossen ist, und der zweite Anschluß mit dem anderen Anschluß beider Spulen verbunden ist. Durch Stromrichtungsumkehr läßt sich bei geeigneter Schaltung der Dioden entweder die eine oder die andere Spule erregen.

Die Ausbildung kann aber im Rahmen der Erfindung auch so getroffen sein, daß der Anker von einem in Achsrichtung des Sondesnkopfes verschiebbaren permanent magnetischen Teil gebildet ist und von einer Spule konzentrisch umgeben ist. Bei einer solchen Ausbildung kann durch einfache Stromrichtungsumkehr an der Zuleitung zur Spule die Bewegungsrichtung des Ankers umgekehrt werden und ein Verschieben der Verankerungsteile in ihre Verankerungslage, oder aus ihrer Verankerungslage zurück in eine eingezogene Lage, erreicht werden.

In allen Fällen ist es besonders vorteilhaft, wenn die Verankerungsteile durch Durchbrechungen des Sondenkopfes hindurch geführt sind un in ihrer Verankerungslage einen Winkel von weniger als 45°, vorzugsweise wenigstens 30° mit der den Sondenkopf tangierenden Radialebene einschließen. Auf diese Weise wird, wie bereits erwähnt, eine große Verankerungsfläche erzielt. In vorteilhafter Weise ist die Ausbildung hiebei so getroffen, daß die Verankerungsteile an ihrer der Achse des Sondenkopfes zugewendeten Seite konvex gekrümmt sind, wodurch das Muskelgewebe nach Art von Widerhaken hintergriffen wird und ein besonders sicherer Halt geboten wird. Vorzugsweise sind wenigstens drei Verankerungsteile über den Umfang des Sondenkopfes verteilt angeordnet, wodurch unabhängig von der Orientierung des Muskelgewebes immer ein sichere Hintergreifen der Muskelfasern ermöglicht wird.

Sollte es erforderlich sein, die bereits im Myocard verankerte Sonde nochmals zu lösen, um sie an einer anderen Stelle festzulegen, so kann neben der Möglichkeit der Verwendung eines Elektromagneten ein weiteres Zugglied vorgesehen sein, mit welchem die Verankerungsteile aus ihrer Verankerungslage zurückziehbar sind.

Die Erfindung ist nachfolgend an Hand von in der Zeichnung dargestellten Ausführungsbeispielen näher erläutert.

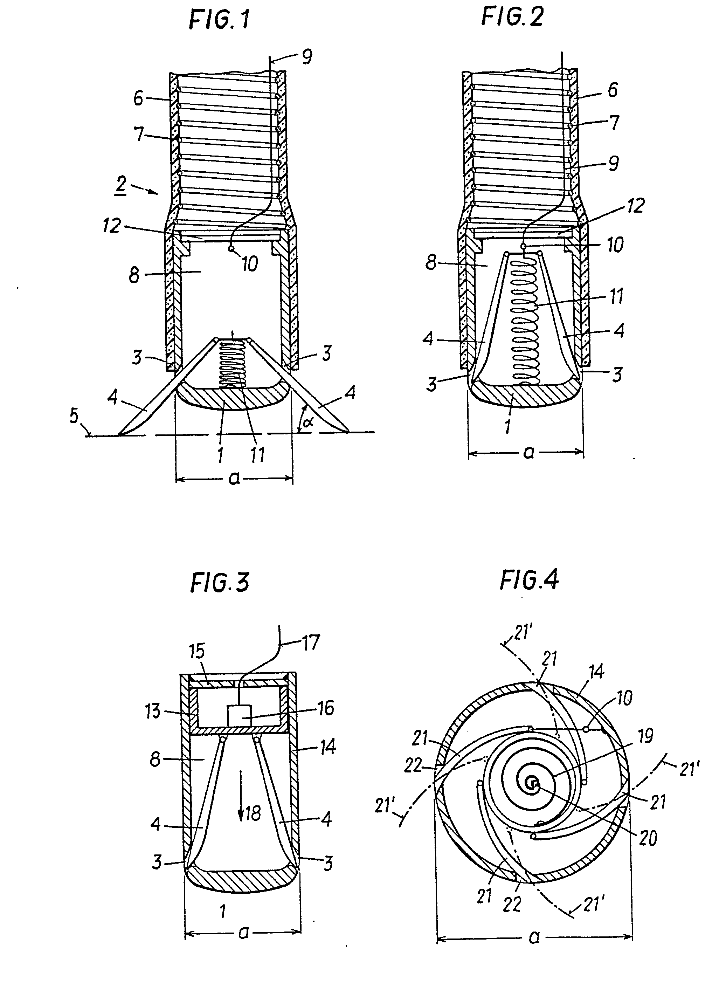

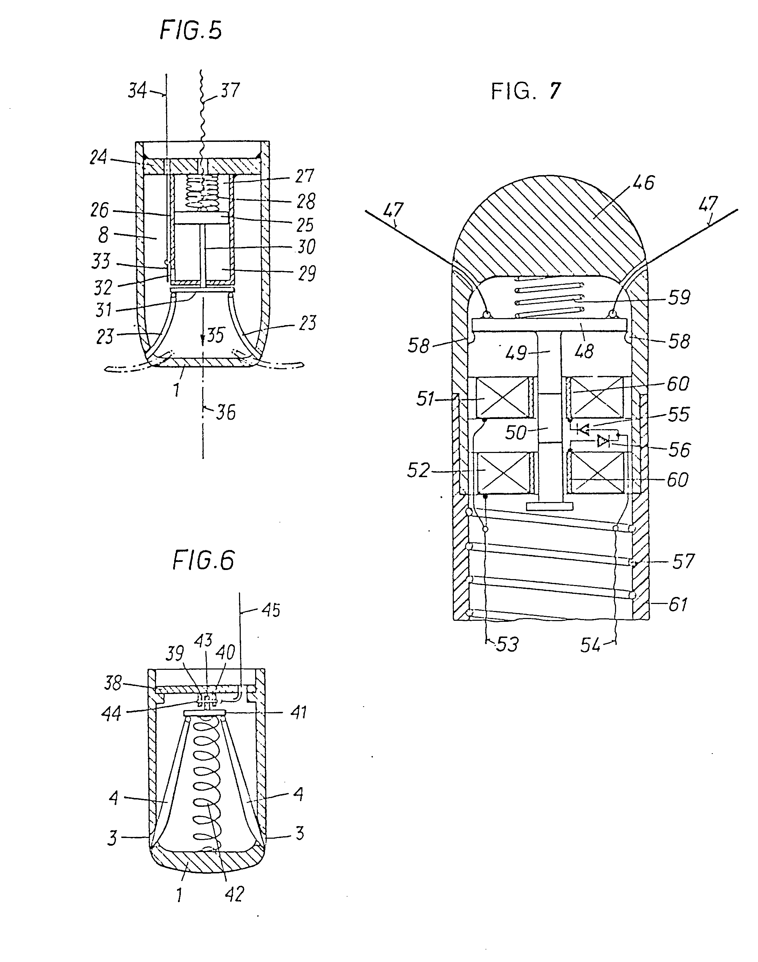

In der Zeichnung zeigt Fig. 1 einen Axialschnitt durch den Sondenkopf mit ausgefahrenen Verankerungsteilen, Fig. 2 den Sondenkopf nach Fig. 1 bei eingezogenen Verankerungsteilen, gleichfalls im Axialschnitt, Fig. 3 einen Axialschnitt durch eine weitere Ausführungsform der erfindungsgemäßen Einrichtung, Fig. 4 einen Schnitt quer zur Achse des Sondenkopfes einer weiteren Ausführungsform der erfindungsgemäßen Einrichtung, Fig. 5 einen Axialschnitt durch einen weiteren erfindungsgemäßen Sondenkopf, Fig. 6 eine weitere Ausführungsform der erfindungsgemäßen Einrichtung im Axialschnitt und Fig. 7 eine Ausbildung mit einem Elektromagneten als Energiewandler.

In Fig. 1 ist der die Elektrode bildende Kopf 1 der Sonde 2 dargestellt. Diese Elektrode besteht üblicherweise aus einer Platiniridiumlegierung. Der Sondenkopf 1 weist Durchbrechungen 3 auf, durch welche Verankerungsteile 4 ausgeschoben werden können. In Fig. 1 sind diese Verankerungsteile in ihrer ausgefahrenen Lage dargestellt, in welcher sie einen Winkel $\alpha$ von etwa 45$^{\circ}$ mit der den Sondenkopf 1 tangierenden Radialebene 5 einschließen. Der Sondenkopf 1 ist in einen üblicherweise aus Silikonkautschuk bestehenden Sondenzuleitungsteil 6 eingeschoben. Innerhalb der Sondenzuleitung 6 verlaufen schraubenlinienförmig aufgewickelte elektrische Leiter 7, welche der Zuleitung des elektrischen Stromes zur vom Sondenkopf 1 gebildeten Elektrode dienen. Das Innere des Sondenkopfes weist einen Hohlraum 8 auf, in welchen ein weiterer elektrischer Leiter 9 hineingeführt ist. Das Ende dieses elektrischen Leiters 9 ist von einer Lötstelle 10 gebildet, welche, wie in Fig. 2 dargestellt, eine Feder 11 in ihrer gespannten Lage halten kann. Der elektrische Leiter 9 ist hiezu an einer Aufhängung 12 gehaltert. Die Verankerungsteile 4 sind mit der ersten an die Lötstelle 10 anschließenden Windung der Feder 11 verbunden und werden von der gespannten Feder 11 in einer Lage gehalten, in welcher sie nicht über den Sondenkopf 1 vorstehen. Die Feder 11 ist mit ihrem der Lötstelle 10 abgewendeten Ende leitend und fest mit dem Sondenkopf 1 verbunden. Von der in Fig. 2 dargestellten Ausgangslage der Verankerungsteile ausgehend kann nun, wenn über die elektrischen Leiter 7 und 9 Strom zugeführt wird, die Lötstelle 10 abgeschmolzen werden, wodurch die gespannte Feder 11 sich entspannen kann und in ihre in Fig. 1 dargestellte Lage gelangen kann. Bei dieser Entspannung der Feder 11 werden die Verankerungsteile rasch in eine über den Außendurchmesser a vorragende Verankungslage gebracht.

In Fig. 3 ist die Ausbildung so getroffen, daß die Verankerungsteile 4 am Boden eines im Hohlraum 8 des Sondenkopfes 1 gleitend verschiebbar gelagerten Kolbens 13 angelenkt sind. Der Kolben 13 selbst ist hohl ausgebildet und
ist in dem durch die Innenwände 14 des Sondenkopfes 1 und
eine mit diesen Innenwänden 14 starr verbundene Abschlußplatte 15 gebildeten Zylinder verschiebbar. Im Hohlraum des
Kolbens 13 ist eine Sprengladung 16 angeordnet und eine Zündleitung 17 erstreckt sich durch die starre Abschlußplatte 15.
Wenn nun diese Sprengladung 16 gezündet wird, wird der
Kolben 13 in dem durch die Wände 14 und die Abschlußplatte
15 gebildeten Zylinder in Richtung des Pfeiles 18 nach abwärts geschleudert, wodurch die Verankerungsteile 4 in ihre
über den Außendurchmesser a des Sondenkopfes 1 vorragende
Verankerungslage gebracht werden.

Bei der Ausbildung nach Fig. 4 sind die Seitenwände 14 des Sondenkopfes ersichtlich, an welchen die Lötstelle 10 festgelegt ist. Die Lötstelle 10 hält eine ebene
Spiralfeder 19 in ihrer gespannten Lage und ist leitend mit
den Wänden 14, welche in der Regel aus dem gleichen Material
wie der Sondenkopf 1 bestehen, verbunden. Das innere Ende 20
der ebenen Spiralfeder 19 ist wiederum mit einem elektrischen
Leiter verbunden und die der Lötstelle 10 benachbarte erste
Windung der ebenen Spiralfeder 19 trägt über ihren Umfang
verteilt vier Verankerungsteile 21. Diese Verankerungsteile
21 sind bei der Darstellung nach Fig. 4 an einem mit der
Feder verbundenen Ring angelenkt und sind leicht gekrümmt.
Die Spitze dieser Verankerungsteile 21 befindet sich in der
gespannten Lage der Feder 19 innerhalb von Durchbrechungen
22 der Seitenwände 14 des Sondenkopfes 1. Wenn nun über die
Seitenwände 14 und das innere Ende der Spiralfeder 19 bei
20 Strom zugeführt wird, kann die Lötstelle 10 schmelzen,
worauf sich die gespannte ebene Spiralfeder 19 entspannt.
Die Verankerungsteile 21 gelangen nach dem Entspannen der
Feder 19 in die durch eine strichlierte Linie angedeutete
Stellung 21', welche der Verankerungslage entspricht, in
welcher die Verankerungsteile über den Durchmesser a des Sondenkopfes vorragen und etwa tangential zu einem gemeinsamen Umhüllungskreis verlaufen. Auf diese Weise läßt sich der Sondenkopf durch Drehen erforderlichenfalls ohne weitere Verletzung des Myocards wieder herausdrehen.

In Fig. 5 ist eine weitere Ausführungsform eines Sondenkopfes 1 dargestellt, bei welchem gekrümmte Verankerungsteile 23 in Durchbrechungen 3 des Sondenkopfes 1 geführt sind. Der Hohlraum 8 des Sondenkopfes 1 ist hier wiederum durch eine Abschlußplatte 24 geschlossen, welche ein Zylinder-Kolben-Aggregat trägt, dessen Kolben mit 25 und dessen Zylinder mit 26 bezeichnet ist. Im Raum 27 zwischen dem Kolben 25 und der Abschlußplatte 24 ist eine Schraubenfeder 28 untergebracht, welche durch einen Gasdruck im Raum 29 an der zur anderen Seite des Kolbens 25 in zusammengepreßter Lage gehalten wird. Die Kolbenstange 30 erstreckt sich durch den Zylinder 26 und trägt ein Querhaupt 31, an welchem die Verankerungsteile 23 angelenkt sind. Der Mantel des Zylinders 26 weist eine Durchbrechung 32 auf, welche von einem Ventil 33 verschlossen ist. Mit dem Ventil 33 ist ein von einer Reißleine 34 gebildetes Betätigungsglied verbunden, welches durch die Abschlußplatte 24 hindurchgeführt ist und durch die Sondenzuleitung, welche in dieser Figur nicht dargestellt ist, nach außen geführt wird. Wenn nun diese Reißleine 34 betätigt wird, wird das Ventil 33 geöffnet und der den Druck der Feder 28 überwindende Gasgegendruck vom Raum 29 des Zylinders 26 aufgehoben. Die Feder 28 drückt den Kolben 25 in Richtung des Pfeiles 35 nach unten, wobei die Verankerungsteile 23 aus dem Sondenkopf 1 ausgefahren werden. Die Verankerungsteile 23 weisen hiebei an ihrer der Achse 36 des Sondenkopfes 1 zugewendeten Seite eine konvexe Krümmung auf und können in der ausgefahrenen Lage das Muskelgewebe hintergreifen. An der dem Raum 27 zugewendeten Seite des Kolbens 25 ist nun bei dieser Ausführungsform ein weiteres Zugglied 37 festgelegt, welches im Inneren des Raumes 27 hinreichend lang ist, um die Ausfahrbewegung der Verankerungsteile 23 nicht zu behindern. Ein derartiges Zugglied 37 ist bei den Ausbildungen nach den Fig. 1 bis 4 nicht dargestellt worden, kann jedoch ohne weiteres auch bei diesen Ausführungen vorgesehen sein. Wenn in der ausgefahrenen Verankerungslage der Verankerungsteile 23 das Zugglied 37 gezogen wird, können die Verankerungsteile 23 wiederum zurück in ihre Ausgangslage gezogen werden, sodaß die Verankerung der Sonde am Ort ihrer Anbringung aufgehoben ist und eine einfache neuerliche Festlegung an einem anderen Ort erfolgen kann. In diesem Falle werden die Verankerungsteile 23 unter dem Druck der durch das Zugglied 37 neuerlich gespannten Feder 28 wiederum nach außen bewegt, wobei diese Verankerung aber in der Regel nicht mehr so sicher ist wie die erstmalige Verankerung, bei welcher die Feder 28 sich schlagartig entspannen konnte.

Bei der Ausbildung nach Fig. 6 ist der Hohlraum 8 des Sondenkopfes 1 von einer Abschlußplatte 38 abgeschlossen, welche in den Hohlraum 8 vorragende Flansche 39, 40 aufweist. Die Verankerungselemente 4 sind den Enden eines T-Balkens 41 angelenkt. An dem T-Balken 41 greift weiters eine Schraubenfeder 42, welche mit ihrem anderen Ende an der Innenseite des Sondenkopfes 1 festgelegt ist, an. Der Steg 43 des T-Balkens 41 weist ebenso wie die Flansche 39 und 40 eine Durchbrechung auf, in welche ein Verriegelungsbolzen 44 eingeschoben ist. Am Verriegelungsbolzen 44 greift ein Zugglied 45 an. Wenn das Zugglied 45 gezogen wird, wird der Verriegelungsbolzen 44 aus den fluchtenden Durchbrechungen des Steges 43 und der Flansche 39 und 40 herausgezogen, wodurch sich die Feder 42 entspannen kann und bei dieser Bewegung die Verankerungsteile 4 in ihre Verankerungslage reißt.

In Fig. 7 ist ein als Elektromagnet ausgebildeter Energiewandler ersichtlich. Mit 46 ist der Sondenkopf bezeichnet, welcher die elektrischen Impulse des Schrittmachers an das Herzmuskelgewebe weiterleitet. Innerhalb des Sondenkopfes sind Verankerungsteile 47 verschieblich geführt, welche in der Zeichnungsfigur in ihrer ausgefahrenen Lage, das heißt in der Verankerungsstellung, gezeigt sind.

Die Verankerungsteile 47 sind gelenkig an einer Ankerplatte 48 festgelegt, und an die Ankerplatte 48 schließt der Anker 49 eines Elektromagneten an. Der Anker 49 kann hiebei aus Kunststoff bestehen und weist einen weichmagnetischen Kern 50 auf, welcher einen Abschnitt des Ankers 49 bildet. Im Inneren des Sondenkopfes 46 sind Spulen 51 und 52 vorgesehen, zu welchen die elektrischen Leitungen 53 und 54 führen. Der Mittenanschluß beider dieser Spulen 51 und 52 steht über Dioden 55 und 56 mit der Zuleitung 54 in Verbindung. Die Dioden 55 und 56 sind hiebei so geschaltet, daß bei einer Polarität der an die Anschlüsse 53 und 54 angeschlossenen Gleichstromquelle jeweils nur eine der beiden Spulen 51 oder 52 erregt wird, da die jeweils andere Diode in dieser Stromrichtung sperrt. Die beiden anderen Anschlüsse der Spulen 51 und 52 sind miteinander verbunden und an die Zuleitung 53 angeschlossen. Die Zuleitung des Reizimpulses erfolgt über einen schraubenlinienförmig aufgewickelten Leiter 57 und auch die Zuleitungen 53 und 54 zu den Spulen des Elektromagneten können, um die Flexibilität der Sondenzuleitung nicht zu gefährden, schraubenlinienförmig aufgewickelt sein.

Die Ankerplatte 48 ist in der in der Zeichnung gezeigten Verankerungsstellung hinter elastisch deformierbaren Vorsprüngen 58 verrastet, sodaß eine unbeabsichtigte Lösung der Verankerungsstellung nicht erfolgen kann. Zum Lösen der Verankerungsteile 47 aus ihrer Verankerungslage, muß die Spule 52 erregt werden, sodaß der ferromagnetische Abschnitt 50 des Ankers 49 in diese Spule 52 gezogen wird, wodurch die Ankerplatte 48 und die daran angelenkten Verankerungsteile 47 wieder zurückgezogen werden. Bei der Darstellung in der Zeichnung ist zur Unterstützung dieser Rückholbewegung der Verankerungsteile 47 eine Feder 59 vorgesehen. Die jeweilige Lage der Verankerungsteile ist am Röntgenschirm nicht unmittelbar verifizierbar, da diese Verankerungsteile in der Regel aus Nylon bestehen und keinen hinlänglichen Kontrast am Röntgenschirm verursachen. Am

0004967

Röntgenschirm ist jedoch die Lage des Sondenkopfes, welcher in der Regel aus einer Platin-Iridium-Legierung besteht, und die Lage des ferromagnetischen Abschnittes 50 relativ zu diesem Sondenkopf, ersichtlich, sodaß die jeweilige Position der Verankerungsteile überprüfbar ist. Wenn auf die Zwischenschaltung der Dioden verzichtet wird und drei Leitungen hinausgeführt werden, von welchen zwei zu jeweils einem Anschluß der beiden Spulen 51 und 52, und die beiden anderen zu den jeweils anderen Anschlüssen der Spulen 51 und 52 führen, kann die jeweilige Position der Verankerungsteile durch Messung der Selbstinduktion der beiden Spulen 51 und 52 ermittelt werden, wobei diejenige Spule den höheren Wert für die Selbstinduktion erreichen wird, welche den ferromagnetischen Abschnitt 50 des Ankers 49 umschließt. Der ferromagnetische Abschnitt 50 aus weichmagnetischem Material kann mit einer gewissen Remanenz ausgebildet sein. Wenn im Inneren der Spule Lagerbüchsen 60 aus Weicheisen vorgesehen sind, kann aufgrund dieser Remanenz eine leicht lösbare Verankerung der jeweiligen Lage der Verankerungsteile erzielt werden.

Bei einer Ausbildung, bei welcher der Anker 49 einen permanent magnetischen Teil aufweist, kann die zweite Spule fortfallen, da es in diesem Fall genügt, die Stromrichtung zu dieser Spule umzukehren.

Die Zuleitung 57 zum Sondenkopf und damit zur Elektrode kann unipolar oder bipolar ausgebildet sein, wobei jeweils eine entsprechende Elektrode vorgesehen sein muß. Die Sondenzuleitung ist mit 61 bezeichnet.

Patentansprüche :

1. Einrichtung zum Festlegen eines Sondenkopfes, insbesondere einer Kardialsonde, bei welcher vorzugsweise von nadelförmigen, gebogenen oder abgewinkelten Teilen gebildete Verankerungsteile mechanisch in ihre über den Außendurchmesser des Sondenkopfes vorragende Verankerungslage am gewünschten Ort bringbar sind, dadurch gekennzeichnet, daß die Verankerungsteile (4; 21;, 23;, 47) mit einem Energiewandler oder einem Energiespeicher (11; 16; 19; 28; 42) zusammenwirken, welcher von einer Stelle außerhalb des Sondenkopfes (2, 46) betätigbar ist.

2. Einrichtung nach Anspruch 1, dadurch gekennzeichnet, daß die Verankerungsteile (4; 21; 23) mit einem Energiespeicher (11;16;19;28;42) zusammenwirken, wobei der vor der Energieabgabe bestehende Zustand des Energiespeichers (11;16;19;28;42) der eingezogenen Ausgangslage der Verankerungsteile (4;21;23) und der nach der Energieabgabe bestehende Zustand des Energiespeichers (11,16,19,28,42) der Verankerungslage derselben entspricht, und daß zur Abgabe der Energie des Energiespeichers (11;16;19;28;42) ein Betätigungsglied (7,9;17;34;45) vorgesehen ist, welches von einer Stelle außerhalb des Sondenkopfes (2) betätigbar ist.

3. Einrichtung nach Anspruch 1 oder 2, dadurch gekennzeichnet, daß der Energiespeicher von einer Sprengpatrone (16) und das Betätigungsglied von einer Zündleitung (17) gebildet sind (Fig. 3).

4. Einrichtung nach Anspruch 1 oder 2, dadurch gekennzeichnet, daß der Energiespeicher von einer Feder (11;19) gebildet ist, welche durch ein lösbares Verriegelungselement (10) in ihrer gespannten Lage festlegbar ist (Fig. 1, 2 und 4).

5. Einrichtung nach Anspruch 4, dadurch gekennzeichnet, daß das Verriegelungselement (44) von einem Haken gebildet ist und daß das Betätigungsglied von einem Zugglied (45) gebildet ist, mit welchem der Haken aus einer Eingriffslage ausklinkbar ist (Fig. 6).

6. Einrichtung nach Anspruch 4, dadurch gekennzeichnet, daß das Verriegelungselement von einem leicht schmelzenden Zugglied, insbesondere einer Lötstelle (10) gebildet ist und daß das Betätigungsglied von den elektrischen Zuleitungen (7,9) für das Schmelzen des Zuggliedes gebildet ist (Fig. 1, 2 und 4).

7. Einrichtung nach einem der Ansprüche 4, 5 oder 6, dadurch gekennzeichnet, daß die Feder von einer Schraubenfeder (11;28;42) gebildet ist.

8. Einrichtung nach einem der Ansprüche 4, 5 oder 6, dadurch gekennzeichnet, daß die Feder von einer ebenen Spiralfeder (14) gebildet ist.

9. Einrichtung nach einem der Ansprüche 7 oder 8, dadurch gekennzeichnet, daß die Verankerungsteile (4;21) an der an das Verriegelungselement anschließenden Windung der Feder (11;19) festgelegt sind (Fig. 1, 2 und 4).

10. Einrichtung nach Anspruch 1 oder 2, dadurch gekennzeichnet, daß der Energiespeicher von einem unter Gasdruck stehenden Zylinder (26) eines Zylinder-Kolbenaggregates (25,26) gebildet ist, dessen Kolben (25) von einer Feder (28) belastet ist, wobei das Betätigungsglied (34) vorzugsweise von einem Zugglied gebildet ist, welches an einem Verschlußteil (32,33) des Zylinders (26) angreift, und die Verankerungsteile (23) mit dem Kolben (25) oder einer Kolbenstange (30) verbunden sind (Fig. 5).

11. Einrichtung nach Anspruch 1, dadurch gekennzeichnet, daß der Energiewandler von einem Elektromagneten (49,50,51) gebildet ist, wobei die Verankerungsteile (47) mit dem Anker (49) des Elektromagneten zusammenwirken und die elektrischen Leitungen (53, 54) zur Versorgung des Elektromagneten in der Sondenzuleitung (61) untergebracht sind.

12. Einrichtung nach Anspruch 11, dadurch gekennzeichnet, daß die Verankerungsteile (47) in ihrer Verankerungslage ebenso wie in ihrer eingezogenen Ausgangslage lösbar verriegelt sind.

0004967

13. Einrichtung nach Anspruch 11 oder 12, dadurch gekennzeichnet, daß die Verankerungsteile (47) federnd in einer der Verankerungsstellung entsprechenden Stellung und/ oder in einer der eingezogenen Ausgangslage entsprechenden Stellung gehalten sind.

14. Einrichtung nach Anspruch 11, 12 oder 13, dadurch gekennzeichnet, daß der Anker (49) einen weichmagnetischen Abschnitt (50) aufweist und daß in Achsrichtung des Sondenkopfes (46) zwei Spulen (51, 52) in Abstand voneinander angeordnet sind, von denen jeweils eine den weichmagnetischen Abschnitt (50) in der eingefahrenen bzw. der ausgefahrenen Stellung der Verankerungsteile (47) umschließt.

15. Einrichtung nach Anspruch 11, 12 oder 13, dadurch gekennzeichnet, daß der Anker von einem in Achsrichtung des Sondenkopfes verschiebbaren permanent magnetischen Teil gebildet ist und von einer Spule konzentrisch umgeben ist.

16. Einrichtung nach einem der Ansprüche 1 bis 15, dadurch gekennzeichnet, daß die Verankerungsteile (4;21;23) durch Durchbrechungen (3;22) des Sondenkopfes (1) hindurch geführt sind und daß sie in ihrer Verankerungslage einen Winkel ( $\alpha$ ) von weniger als 45°, vorzugsweise jedoch größer als 30° mit der den Sondenkopf (1) tangierenden Radialebene (5) einschließen.

17. Einrichtung nach einem der Ansprüche 1 bis 16, dadurch gekennzeichnet, daß die Verankerungsteile (4;23) im Axialschnitt betrachtet, an ihrer der Achse des Sondenkopfes (1) zugewendeten Seite konvex gekrümmt sind.

18. Einrichtung nach einem der Ansprüche 1 bis 17, dadurch gekennzeichnet, daß wenigstens drei Verankerungsteile (4;21;23) über den Umfang des Sondenkopfes (1) verteilt angeordnet sind.

19. Einrichtung nach einem der Ansprüche 1 bis 18, dadurch gekennzeichnet, daß die Verankerungsteile (21) in ihrer Projektion auf eine Radialebene gekrümmt sind, wobei der der Achse der Sonde zugewendete Teil der Verankerungsteile (21) konkav gekrümmt ist (Fig. 4).

- 4 -

20. Einrichtung nach einem der Ansprüche 1 bis 19, dadurch gekennzeichnet, daß ein weiteres Zugglied (27) vorgesehen ist, mit welchem die Verankerungsteile (23) aus ihrer Verankerungslage zurückziehbar sind (Fig. 5).

FIG.1

FIG.2

FIG.3

FIG.4

FIG.5

FIG. 7

FIG.6